# EUROPEAN PATENT SPECIFICATION

(11) **EP 0 456 748 B1**
(45) Date of publication and mention of the grant of the patent: **28.08.1996**
(21) Application number: 90903256.7
(22) Date of filing: 26.01.1990
(51) Int. Cl.: A61M 25/00

(54) **A SINGLE-LUMEN, BIDIRECTIONAL, CHECK VALVE CATHETER**
EINLUMIGER ZWEIWEGKATHETER MIT RÜCKSCHLAGVENTIL
CATHETER A VANNE DE CONTROLE BIDIRECTIONNELLE, A UN SEUL PASSAGE

(30) Priority: 02.02.1989 US 305971
(43) Date of publication of application: 21.11.1991
(73) Proprietor: REGENTS OF THE UNIVERSITY OF MINNESOTA, Minneapolis, Minnesota 55455 (US)
(72) Inventor: WIGNESS, Bruce, D., Minneapolis, MN 55404 (US); DORMAN, Frank, D., Minneapolis, MN 55422 (US)
(74) Representative: Orr, William McLean
(86) International application number: US9000588
(87) International publication number: WO9009204

(56) References cited:
- DE-C- 336 530
- FR-A- 2 144 806
- US-A- 2 747 608
- US-A- 3 995 617
- US-A- 4 657 536
- US-A- 4 701 166
- US-A- 4 705 501
- US-A- 4 753 640

## Description

### Background of the Invention

The present invention relates to a single lumen catheter having a bidirectional check valve proximate the tip for controlling fluid flow into and out of the catheter.

There are many medical requirements for chronic vascular access including neonatal umbilical vein cannulation, hyperalimentation, chemotherapy, permanent intravascular site for portable drug infusion devices, hemodialysis, plasmapheresis, and, repeated blood sampling. These procedures are performed by using either transcutaneous or totally implanted intravascular catheters.

Examples of transcutaneous catheters include the Broviak and Hickman central venous catheters (Evermed, Kirkland, WA) as well as many similar products generically termed subclavian catheters. Catheters in this category may be generally described as being plastic or rubber tubes with female luer fittings at one end. Catheter patency is maintained by filling the catheter lumen with a solution containing an anticoagulant (heparin) between uses. Since the anticoagulant consistently diffuses from the lumen and into the bloodstream, the catheter has to be refilled at 2-3 day intervals in order to prevent a clot from forming at the distal tip.

The Shiley Vas-Cath (Shiley, Inc., Irvine, CA) is a transcutaneous subclavian catheter which differs from subclavian catheters in that it has a removable lining. In the event that the catheter clots, the lining, which contains the clot, is extracted from the outer sheath and replaced.

Another solution to catheter lumen clotting involves the insertion of a solid flexible plastic rod, called an obturator, into the catheter lumen between uses. The obturator completely occupies the catheter lumen and therefore prevents diffusion of blood components and subsequent formation of a clot.

An alternate obturated design is the Wigness/Anderson U.S. Patent No. 4,705,501 entitled, "Bidirectional Antireflux Vascular Access System", which features a self-contained flexible obturator which resembles a bladder which runs the length of the catheter lumen and is not removed between uses, but is instead shifted between the "active" state (where the lumen is open) and the "dormant" state (where the lumen is occluded by the obturator).

The advantages offered by an intravascular catheter in which the lumen may be closed and therefore isolated from the blood stream when it is not in use are:
1. It is resistant to occlusion from thrombosis;
2. It decreases the risk of air embolism; and
3. It requires less maintenance (e.g., heparinization and irrigation).

Examples of totally implantable catheters, generically referred to as "ports", include the "Infuse-A-Port" (Shiley-Infusaid, Inc., Norwood, MA), the "Q-Port" (Quinton Instrument Co., Seattle, WA), and the "Port-A-Cath" (Pharmacia-Deltec, Inc., St. Paul, MN). Ports are similar to subclavian catheters except that the female luer fitting is replaced by a metal or plastic manifold which houses the fluid conduits between a rubber access septum and the catheter connector. Although the catheters are routinely filled with a heparin solution between uses, if the time interval between catheter uses is relatively long (weeks instead of days), each therapy session is initiated by blowing a small clot into the vasculature.

Advantages offered by a catheter which furthermore is totally implantable are:
1. Longer useful life due to reduced risk of infection;
2. Fewer recannulations and therefore less discomfort to the patient; and
3. More patient freedom (the patient may shower, bathe or swim without having to perform special catheter maintenance).

Designs featuring removable obturators or replaceable linings cannot be modified to be totally implantable by simply attaching them to a port, since that would require that a relatively large, solid element be passed through the skin, subcutaneous tissue and catheter septum with each use.

In order to achieve total implantability of the previously described Wigness/Anderson obturated catheter, it is attached either to a special manifold (Wigness/Dorman patent application dated October 6, 1987, U.S. Serial No. 105,740, entitled, "Implantable Intravascular Access System") or two septa--one for the functional lumen, and one which is used for shifting the obturator.

Diffusion or aspiration of blood into the subclavian-type catheters may be prevented by adding a check valve to their intravascular tips. The Dorman U.S. Patent No. 4,657,536 entitled, "Check Valve Catheter" describes a sleeve type one-way check valve design for this purpose. While this design prevents blood components from clotting the tip, it does not allow withdrawal of a blood sample--a maneuver that is sometimes desirable and commonly carried out with subclavian catheters and ports. A commercially available port with a one-way check valve at the tip is the "Implantofix" (Burron Medical Inc., Bethlehem, PA).

One commercially available catheter featuring a slit valve at the tip which allows both aspiration of blood and infusion of fluids and yet precludes the diffusion of blood components into the lumen between therapy sessions is the Groshong Catheter (Catheter Technology Corporation, Salt Lake City, UT). This catheter is available both as a transcutaneous appliance and also as a port.

It is also known from FR-A-2144806 to provide a bi-directional check valve catheter tip which comprises an elongated flexible tubular member having a distal end and a proximal end, a lumen defined by the tubular member and having port means in a wall of the lumen for transferring fluid to and from the lumen, and first unidirectional check valve means responsive to changes in fluid pressure for allowing fluid flow through the port means into the lumen and second unidirectional check valve means responsive to changes in fluid pressure for allowing fluid flow through the port means from the lumen. A cavity of a substantial size is located distally of the outlet check valve means. This arrangement is prone to the risk of residual blood components clotting in the lumen of the catheter thereby impairing performance. Furthermore, the device of FR-A-2144806 is adapted for use with a pump for cardio synchronised pumping of blood, and is incapable of introducing, or withdrawing, the fluid to or from the blood stream.

The invention seeks to improve known catheter devices by providing an inlet check valve means which offers resistance to the flow of fluid into the lumen and an exit valve means which offers resistance to the flow of fluid out of the lumen, thereby preventing the flow of fluid through either port means until such flow is desired, such as when aspirating or injecting fluid into or out of the lumen of the catheter tip.

According to the invention there is provided a bi-directional check valve catheter tip for intravascular placement in a living body and comprising:
an elongated bio-compatible flexible tubular member having a distal end and a proximal end;
a central lumen defined by the tubular member and having inlet port means and outlet port means defined in a wall of the tubular member which surrounds the lumen; and,
first unidirectional check valve means responsive to differential fluid pressure action applied thereto in order to allow fluid flow through the inlet port means and into the lumen, and second unidirectional check valve means responsive to differential fluid pressure action applied thereto in order to allow fluid flow through the outlet port means from the lumen:
characterised in that the outlet port means is located distally of the inlet port means so that saline solution introduced from said proximal end of the tubular member and into the lumen can exit via the outlet port means in order to totally rinse the lumen of blood components present in the lumen; and in that the first and second check valve means normally maintain the respective port means closed in the absence of fluid differential pressure action being applied to each check valve means.

Thus, in a bi-directional check valve catheter tip according to the invention, the first unidirectional check valve means functions as an inlet check valve means offering resistance to the flow of fluid into the lumen and the second unidirectional check valve means functions as exist check valve means offering resistance to the flow of fluid out of the lumen, thereby preventing the flow of fluid into or out of the lumen until such flow is desired. Furthermore, to achieve such required flow, substantial pressure differential (negative or positive) will be required, unlike the arrangement in the French patent reference where simple elastic tongues are provided which function as flap valves which will easily open and which are deliberately designed to provide minimal resistance to the flow of blood in the desired direction when the catheter tip is employed to facilitate cardiac action in cases of contractile incompetence.

Furthermore, the bi-directional check valve catheter of the invention has the outlet port means located distally of the inlet port means to enable saline solution to be infused into the lumen of the catheter from the proximal end of the tubular member in order to rinse totally the lumen of any blood components. This greatly reduces the risk of blood clotting in the lumen of the catheter.

One embodiment of the present invention is an intravascular single lumen catheter featuring inlet and outlet check valve action which is controlled by separate valves. The inlet check valve is a tubular rubber element, located in the catheter lumen, which is normally closed against a fixed geometry inlet orifice. Blood sampling is achieved by aspirating, on a proximal end of the catheter, with enough force to create a pressure drop between the blood stream and the catheter lumen which is sufficient to overcome the elastic forces within the wall of the tubular valve, causing the valve to collapse open, thus permitting blood withdrawal. The outlet check valve is an elastic rubber sleeve which is normally closed against a fixed geometry outlet orifice from the vascular side of the catheter. Fluid infusion into the catheter lumen increases the lumen pressure until it is substantially higher than the elastic forces within the valve sleeve plus intravascular blood pressure, at that point fluid is injected via the outlet port through a variable annular passageway created between the sleeve and the vascular side of the catheter.

A particular advantage of one embodiment of the present invention is that it provides an intravascular catheter design which permits long-term placement in the blood stream without requiring routine anticoagulant flushing in order to prevent thrombus formation within the catheter lumen. This is achieved by controlling communication between the catheter lumen and the bloodstream with normally closed check valves.

These and various other advantages and features of novelty which characterize the invention are pointed out with particularity in the claims annexed hereto and forming a part hereof. However, for a better understanding of the invention, its advantages and objects obtained by its use, reference should be made to the drawings which form a further part hereof, and to the accompanying descriptive matter, in which there is illustrated and described a preferred embodiment of the invention.

### Brief Description of the Drawings

In the drawings, wherein like reference numerals indicate corresponding parts throughout the several views,
Fig. 1A is a diagrammatic view of a vascular access system utilizing a catheter having a bidirectional check valve utilizing a syringe-stopcock subassembly, as typically used on transcutaneously placed catheters, in accordance with the principles of the present invention;
Fig. 1B is a view similar to Fig. 1A illustrating a vascular access system wherein the syringe-stopcock assembly is replaced by an implantable manifold subassembly;
Fig. 2 is a cross-sectional view of a catheter tip portion incorporating an embodiment of a bidirectional check valve assembly in accordance with the principles of the present invention;
Fig. 3A is a view similar to Fig. 2 illustrating the inlet check valve in an open state such as when aspirating blood into the lumen of the catheter;
Fig. 3B is a view similar to Fig. 2 illustrating the outlet check valve in an open state such as when injecting fluid from the catheter lumen into the body;
Fig. 4A is a cross-sectional view taken generally along line 4A in Fig. 2 illustrating the inlet check valve in its normally closed state;
Fig. 4B is a cross-sectional view taken generally along line 4B of Fig. 3A illustrating the inlet check valve in its opened state;
Fig. 5A is a cross-sectional view taken generally along 5A of Fig. 2 illustrating the outlet check valve in its normally closed state;
Fig. 5B is a cross-sectional view taken generally along 5B of Fig. 3B illustrating the outlet check valve in its opened state;
Fig. 6 is a view similar to Fig. 2 illustrating an alternative embodiment of the exit valve tubular member including reinforcement material.

### Detailed Description of the Preferred Embodiment

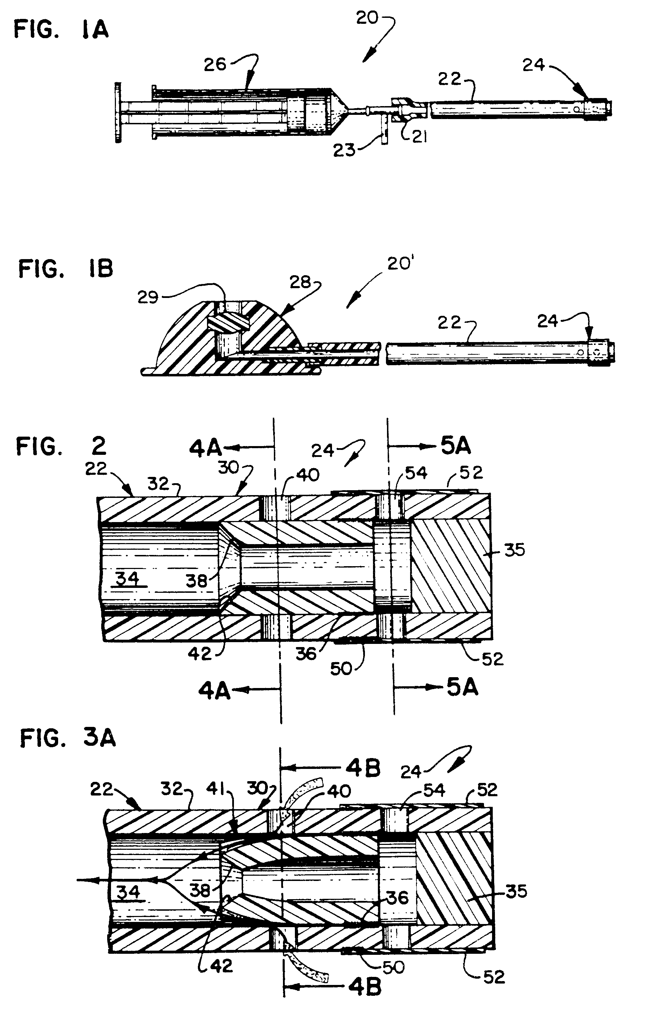

Referring now to the drawings there is shown in Fig. 1A a vascular access system 20 utilizing a single lumen catheter 22 having a bidirectional check valve assembly 24 in accordance with the principles of the present invention. The vascular access system 20 shown in Fig. 1 typically uses transcutaneously placed catheters. Fluid injection and aspiration is shown being accomplished by the use of a syringe-stopcock subassembly 26 which is suitably connected to the exposed end of the catheter 22. Fig. 1B illustrates a vascular access system 20' which incorporates an implantable manifold subassembly 28 such that the vascular access system 20' is totally implantable in the body. The manifold subassembly 28 includes a self-sealing septum 29 through which a needle can be inserted to inject fluid into the manifold subassembly 28. The bidirectional check valve catheter of the present invention can be used with either of the vascular access systems shown in Figs. 1A,B.

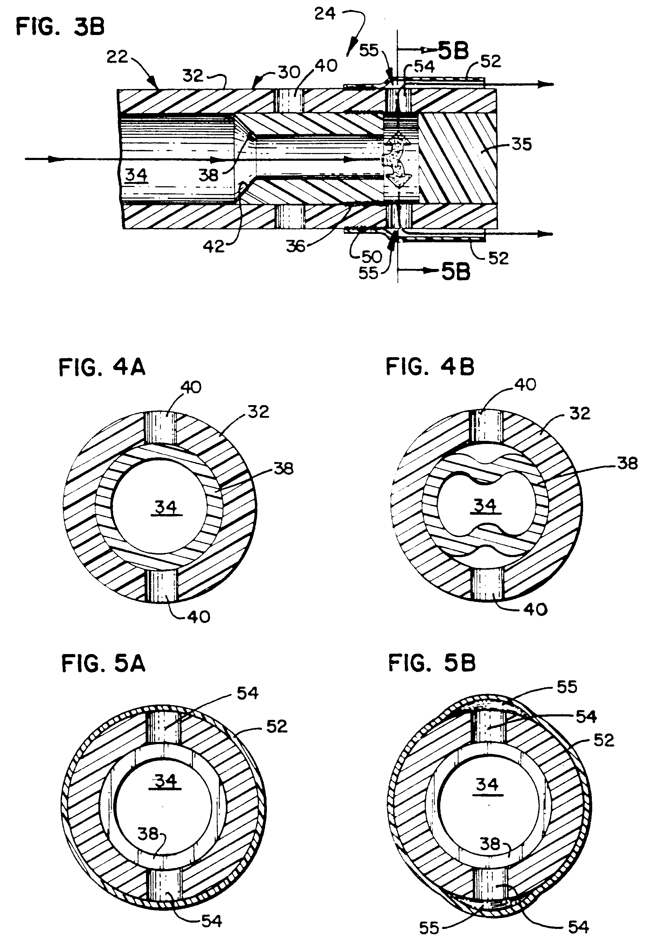

Referring now to Fig. 2, there is illustrated a preferred embodiment of a bidirectional check valve assembly 24 in accordance with the principles of the present invention. A distal tip portion 30 of the catheter 22 is illustrated. The catheter 22 includes a hollow tubular wall member 32 defining a central lumen 34. The distal end of the catheter 22 is closed by a plug member 35. Bonded at location 36 to an inner surface of the central lumen 34 is a hollow tubular member 38 which is also referred to herein as an inlet check valve. The tubular member 38 covers two fixed geometry, diametrically opposed inlet ports 40 in the tubular wall 32 of the catheter 22. A proximal end 42 of the tubular member 38 is tapered as illustrated. In its normal state, the tubular member 38 covers the inlet ports 40 to form a substantially fluid-tight seal so as to prevent body fluid from entering into the catheter 22. However, when a syringe or other device is used to create a decrease in fluid pressure inside the central lumen 34, the tubular member 38 partially collapses to open the inlet ports 40 and allows fluid to flow from the body and into the lumen 34 as generally illustrated in figure 3A. Figs. 4A,B respectively illustrate the tubular member 38 in its normal state and when it is partially collapsed due to a decrease in fluid pressure in the lumen 34 such as when aspirating fluids from the body. As illustrated in Fig. 4B, when the tubular member 38 partially collapses, it forms passageways 41 from the lumen 34 to the inlet ports 40.

As further illustrated in Fig. 2, bonded at 50 to an exterior surface of the catheter wall member 32, is an elastic sleeve 52 which is also referred to herein as an exit check valve. It will be appreciated that the sleeve might be made out of silicone rubber or any number of suitable materials. The elastic sleeve 52 covers two fixed geometry, diametrically opposed exit ports 54 in the tubular wall 32 of the catheter 22. The exit ports 54 are axially removed from the inlet ports 40 toward the distal end of the catheter 22. In one embodiment, in its normal state, the elastic sleeve 52 covers the exit ports 54 to form a substantially fluid-tight seal so as to prevent body fluid from entering the lumen 34 of the catheter. When a syringe or other device is used to create an increase in pressure inside the central lumen 34, the elastic sheath 52 partially expands to open the exit ports 54 and allows fluid to flow from the lumen 34 into the body as generally illustrated in Fig. 3B. Figs. 5A,B illustrate the elastic sheath 52 in its normal state and when it is partially expanded due to an increase in fluid pressure in the lumen 34 such as when injecting fluids into the body. In yet other embodiments, wherein fluid is continuously being delivered to the body, the elastic sleeve 52 might remain partially expanded. As illustrated in Fig. 5B, when the elastic sleeve 52 is partially expanded, it forms passageways 55 from the lumen 34 and the exit ports 54 into the body 55.

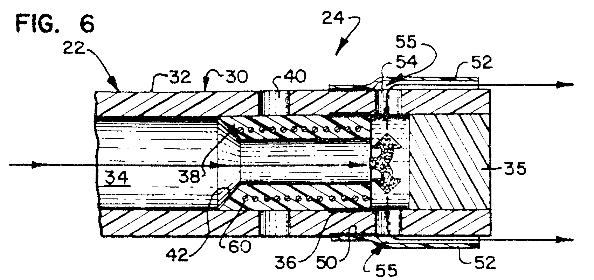

Illustrated in Fig. 6 is an alternate embodiment of the present invention wherein the tubular member 38 is reinforced by a flexible, nonelastic material or fabric 60 embedded in the tubular member 38 and wound in a coil along the length of the tubular member 38. The nonelastic material might be made of any number of materials such as polyurethane, etc. The reinforcement material 60 allows the tubular member 38 to collapse open and perform as an inlet check valve as generally discussed above; however, the reinforcement material 60 does not permit significant expansion of the tubular member 38. It will be appreciated, that in alternate embodiments, the tubular member 38 might be made of a nonelastic flexible sleeve or be constructed as a mesh or any number of other ways.

Operation of vascular access systems incorporating catheters with bidirectional check valve assemblies in accordance with the principles of the present invention will now be described. With respect to the first embodiment shown in Fig. 2, when the catheter 22 is not in use, the valves of the bidirectional check valve assembly 24 are in their normally closed state as generally illustrated in Fig. 2. There is no fluid communication between the bloodstream and the catheter lumen 34 and a female luer fitting 21 at a proximal end of a percutaneously placed catheter is occluded by a stopcock 23. It will be appreciated that the proximal end of the catheter 22 might be occluded by any number of methods other than a stopcock such as a punctureable septum or a solid plug. Aspiration of a blood sample is achieved by installing an empty syringe 26 on the female luer fitting 21. Withdrawal of the syringe barrel causes a decrease in pressure within the catheter lumen 34. When the pressure drop from the blood vessel to the catheter lumen exceeds the elastic forces within the wall of the tubular compression valve member 38, the valve 38 is forced open and blood is aspirated via the fixed geometry inlet port 40 and the variable annular inlet passageway 41 into the catheter lumen 34 and onto the syringe 26 via the catheter 22. At the end of a blood withdrawal procedure, the catheter 22 is normally flushed with a normal saline solution (0.9% NaCl) by replacing the blood collecting syringe 26 with a syringe 26 containing normal saline and injecting the contents into the catheter. The first creates a pressure increase within the catheter lumen 34 until the difference between the lumen pressure and the vascular blood pressure exceeds the elastic forces within the exit port check valve sleeve 52, at which time, normal saline is forced via the fixed geometry exit port 54 through the variable annular exit passageway 55 into the bloodstream. In this manner, sufficient normal saline volume is infused in order to totally rinse the lumen 34 of any blood components.

It will be appreciated that the sequence of events for the totally implantable vascular access system as shown in Fig. 1B is similar to the percutaneous vascular access system design shown in Fig. 1A except that instead of connecting a syringe 26 directly to the female luer fitting 21 of the catheter 22, the syringe 26 is connected to a hypodermic needle which is in turn inserted through the skin and the access septum of the manifold 28 and into the manifold's plenum which in turn communicates with the catheter lumen 34.

It is to be understood, however, that even though numerous characteristics and advantages of the present invention have been set forth in the foregoing description, together with details of the structure and function of the invention, the disclosure is illustrative only, and changes may be made in detail, especially in matters of shape, size and arrangement of parts within the principles of the invention to the full extent indicated by the broad general meaning of the terms in which the appended claims are expressed.

## Claims

1. A bi-directional check valve catheter tip for introvascular placement in a living body and comprising:
an elongated bio-compatible flexible tubular member (22) having a distal end (35) and a proximal end (42);
a central lumen (34) defined by the tubular member (22) and having inlet port means (40) and outlet port means (54) defined in a wall (32) of the tubular member which surrounds the lumen (34); and,
first unidirectional check valve means (38) responsive to differential fluid pressure action applied thereto in order to allow fluid flow through the inlet port means (40) and into the lumen (34), and second unidirectional check valve means (52) responsive to differential fluid pressure action applied thereto in order to allow fluid flow through the outlet port means (54) from the lumen (34):
characterised in that the outlet port means (54) is located distally of the inlet port means (40) so that saline solution introduced from said proximal end (42) of the tubular member (22) and into the lumen (34) can exit via the outlet port means (54) in order to totally rinse the lumen (34) of blood components present in the lumen; and in that the first and second check valve means (38, 52) normally maintain the respective port means (40, 54) closed in the absence of fluid differential pressure action being applied to each check valve means.

2. A bi-directional catheter tip according to claim 1, characterised in that the first check valve means (38) includes tubular compression means attached to an interior of the tubular member (22) and normally closing the inlet port means (40) for allowing fluid to flow into the lumen (34) upon detection of decreased pressure such as when aspirating blood from a body into the lumen, and in that the second check valve means (54) includes elastic sleeve means attached to an exterior of the tubular member (22) and normally closing the exit port means (54) for allowing fluid to flow from the lumen upon detection of increased pressure such as when injecting drugs from the lumen to the body.

3. A bi-directional catheter tip according to claim 2, characterised in that the tubular compression means (38) includes reinforcement material (60) embedded therein.

4. A bi-directional check valve catheter tip according to claim 1, characterised in that the proximal end of the flexible tubular member (22) is adapted for interconnection to a fluid injection / aspiration device.

5. A vascular access system including a catheter tip according to claim 4, characterised by fluid aspiration / injection means (26) interconnected to the proximal end (21) of the tubular member (22) and in fluid communication with the lumen (34) for aspirating and injecting fluid into and out of the lumen.

## Patentansprüche

1. Eine Zweiwegkatheterspitze mit Rückschlagventil zum introvaskulären Plazieren in einen lebenden Körper und umfassend:
ein verlängertes biokompatibles flexibles röhrenförmiges Element (22) mit einem distalen Ende (35) und einem proximalen Ende (42):
ein zentrales Lumen (34), das durch das röhrenförmige Element (22) definiert ist, und ein Einlaßöffnungsmittel (40) und ein Auslaßöffnungsmittel (54) aufweist, die in einer Wand (32) des röhrenförmigen Elementes definiert sind, die das Lumen (34) umgibt; und,
ein erstes Einwegrückschlagventilmittel (38), das auf darauf aufgebrachte Differentialfluiddruckeinwirkung anspricht, um Fluidstrom durch das Einlaßöffnungsmittel (40) und in das Lumen (34) zu erlauben, und ein zweites Einwegrückschlagventilmittel (52), das auf darauf aufgebrachte Differentialfluiddruckeinwirkung anspricht, um Fluidstrom durch das Auslaßöffnungsmittel (54) aus dem Lumem (34) zu erlauben:
dadurch gekennzeichnet, daß das Auslaßöffnungsmittel (54) distal vom Einlaßöffnungsmittel (40) angeordnet ist, so daß Salzlösung, die von besagtem proximalen Ende (42) des röhren förmigen Elementes (22) und in das Lumen (34) eingeführt wird, über das Auslaßöffnungsmittel (54) austreten kann, um das Lumen (34) vollständig von im Lumen vorhandenen Blutkomponenten auszuspülen; und dadurch, daß das erste und zweite Rückschlagventilmittel (38, 52) normalerweise die entsprechenden Öffnungsmittel (40, 54) in der Abwesenheit von Fluiddifferentialdruckeinwirkung, die auf jedes Rückschlagventilmittel aufgebracht wird, geschlossen halten.

2. Eine Zweiwegkatheterspitze nach Anspruch 1, dadurch gekennzeichnet, daß das erste Rückschlagventilmittel (38) ein röhrenförmiges Kompressionsmittel einschließt, das an einer In nenfläche des röhrenförmigen Elementes (22) befestigt ist und normalerweise das Einlaßöffnungsmittel (40) verschließt, um zu erlauben, daß nach Feststelltung von verringertem Druck, wie z.B. wenn Blut aus einem Körper in das Lumen gesaugt wird, Fluid in das Lumen (34) strömt, und dadurch, daß das zweite Rückschlagventilmittel (54) ein elastisches Hülsenmittel einschließt, das an einer Außenfläche des röhrenförmigen Elementes (22) befestigt ist, und normalerweise das Austrittsöffnungsmittel (54) verschließt, um zu erlauben, daß nach Feststellung erhöhten Drucks, wie z. B. wenn Arzneimittel aus dem Lumen in den Körper injiziert werden, Fluid aus dem Lumen strömt.

3. Eine Zweiwegkatheterspitze nach Anspruch 2, dadurch gekennzeichnet, daß das röhrenförmige Kompressionsmittel (38) darin eingebettetes Verstärkungsmaterial (60) einschließt.

4. Eine Zweiwegkatheterspitze mit Rückschlagventil nach Anspruch 1, dadurch gekennzeichnet, daß das proximale Ende des flexiblen röhrenförmigen Elementes (22) angepaßt ist für Verbindung mit einer Fluidinjektions-/aspirationsvorrichtung.

5. Ein vaskuläres Zugangssystem, das eine Katheterspitze nach Anspruch 4 einschließt, gekennzeichnet durch Fluidaspirations-/injektionsmittel (26), das mit dem proximalen Ende (21) des röhrenförmigen Elementes (22) verbunden ist und in Fluidverbindung mit dem Lumen (34) steht zum Aspirieren und Injizieren von Flüssigkeit in das Lumen und aus dem Lumen.

## Revendications

1. Embout de cathéter à vanne de contrôle bidirectionnelle pour implantation intravasculaire dans un corps vivant et comprenant :
un élément (22) flexible, bio-compatible, allongé, ayant une extrémité distale (35) et une extrémité proximale (42);
une lumière centrale (34) définie par l'élément tubulaire (22) et ayant des orifices d'entrée (40) et des orifices de sortie (54) définis dans une paroi (32) de l'élément tubulaire qui entoure la lumière (34); et,
des premiers moyens formant vanne de contrôle unidirectionnelle (38) sensibles à l'action de la pression différentielle d'un fluide qui leur est appliquée, de façon à permettre l'écoulement d'un fluide à travers les orifices d'entrée (40) et à l'intérieur de la lumière (34), et des seconds moyens formant vanne de contrôle unidirectionnelle (52) sensibles à l'action de la pression différentielle d'un fluide qui leur est appliquée, de façon à permettre l'écoulement d'un fluide à travers les orifices d'entrée (54), depuis la lumière (34) :
caractérisé en ce que les orifices de sortie (54) sont situés de façon distale par rapport aux orifices d'entrée (40), de sorte qu'une solution saline introduite depuis ladite extrémité proximale (42) de l'élément tubulaire (22) et à l'intérieur de la lumière (34) peut sortir via les orifices de sortie (54), de façon à rincer totalement la lumière (34) des composants sanguins présents dans la lumière ; et en ce que les premiers et seconds moyens formant vannes de contrôle (38, 52) maintiennent normalement les orifices respectifs (40, 54) fermés, en l'absence de l'action d'une pression différentielle d'un fluide appliquée à chaque moyen formant vanne de contrôle.

2. Embout de cathéter bidirectionnel selon la revendication 1, caractérisé en ce que les premiers moyens formant vanne de contrôle (38) inclut des moyens de compression tubulaire fixés à l'intérieur de l'élément tubulaire (22) et fermant normalement les orifices d'entrée (40), pour permettre au fluide de s'écouler à l'intérieur de la lumière (34) lors de la détection d'une diminution de pression telle que celle qui survient lors de l'aspiration du sang depuis le corps jusqu'à l'intérieur de la lumière, et en ce que les seconds moyens formant vanne de contrôle (54) incluent des moyens formant manchon élastique fixés à l'extérieur de l'élément tubulaire (22) et fermant normalement les orifices de sortie (54) pour permettre au fluide de s'écouler depuis la lumière lors de la détection d'une augmentation de pression telle que celle qui survient lorsque l'on injecte des médicaments depuis la lumière dans le corps.

3. Embout de cathéter bidirectionnel selon la revendication 2, caractérisé en ce que les moyens de compression tubulaire (38) incluent un matériau de renforcement (60) inséré dans lesdits moyens de compression.

4. Embout de cathéter bidirectionnel selon la revendication 1, caractérisé en ce que l'extrémité proximale de l'élément tubulaire flexible (22) est adapté pour une interconnexion à un dispositif d'injection/aspiration de fluide.

5. Système d'accès vasculaire incluant un embout de cathéter selon la revendication 4, caractérisé par des moyens d'aspiration/injection de fluide (26) interconnectés à l'extrémité proximale (21 ) de l'élément tubulaire (22) et en communication de fluide avec la lumière (34) pour l'aspiration et l'injection de fluide à l'intérieur et à l'extérieur de la lumière.
